(19) Europäisches Patentamt
European Patent Office
Office européen des brevets

(11) **EP 1 558 242 B1**

(12) **EUROPEAN PATENT SPECIFICATION**

(45) Date of publication and mention
of the grant of the patent:
**24.12.2008 Bulletin 2008/52**

(51) Int Cl.:
*A61K 31/337* (2006.01)     *A61K 31/52* (2006.01)
*A61P 35/00* (2006.01)

(21) Application number: **03810527.6**

(22) Date of filing: **05.11.2003**

(86) International application number:
**PCT/GB2003/004780**

(87) International publication number:
**WO 2004/041268 (21.05.2004 Gazette 2004/21)**

(54) **COMBINATION COMPRISING DOCETAXEL AND A CDK INHIBITOR**

KOMBINATION AUS DOCETAXEL UND EINEM CDK-HEMMER

COMBINAISON COMPRENANT DU DOCETAXEL ET UN INHIBITEUR DE CDK

(84) Designated Contracting States:
**AT BE BG CH CY CZ DE DK EE ES FI FR GB GR
HU IE IT LI LU MC NL PT RO SE SI SK TR**

(30) Priority: **06.11.2002 GB 0225872**
**11.08.2003 GB 0318813**

(43) Date of publication of application:
**03.08.2005 Bulletin 2005/31**

(73) Proprietor: **Cyclacel Limited**
**London N1 7JQ (GB)**

(72) Inventor: **Giannella-Borradori, Athos,**
**Cyclacel Limited**
**Dundee DD1 5JJ (GB)**

(74) Representative: **Clyde-Watson, Zöe et al**
**D Young & Co**
**120 Holborn**
**London EC1N 2DY (GB)**

(56) References cited:
WO-A-02/46182          WO-A-02/076484
WO-A-03/039536          US-A1- 2003 125 374

• MOTWANI M V ET AL: "DOCETAXEL AND NAVELBINE INDUCED APOPTOSIS IS ENHANCED BY FLAVOPIRIDOL (FLAVO) IN BREAST CANCER CELLS AND IS SEQUENCE DEPENDENT" PROCEEDINGS OF THE ANNUAL MEETING OF THE AMERICAN ASSOCIATION FOR CANCER RESEARCH, NEW YORK, NY, US, vol. 41, March 2000 (2000-03), page 143,AN912 XP008008541 ISSN: 0197-016X
• MOTWANI M ET AL: "SEQUENTIAL DEPENDENT ENHANCEMENT OF CASPASE ACTIVATION AND APOPTOSIS BY FLAVOPIRIDOL ON PACLITAXEL-TREATED HUMAN GASTRIC AND BREAST CANCER CELLS" CLINICAL CANCER RESEARCH, THE AMERICAN ASSOCIATION FOR CANCER RESEARCH, US, vol. 5, no. 7, July 1996 (1996-07), pages 1876-1883, XP001113185 ISSN: 1078-0432
• MOTWANI MONICA ET AL: "Flavopiridol enhances the effect of docetaxel in vitro and in vivo in human gastric cancer cells." MOLECULAR CANCER THERAPEUTICS, vol. 2, no. 6, June 2003 (2003-06), pages 549-555, XP009025301 ISSN: 1535-7163 (ISSN print)

**Description**

**FIELD OF THE INVENTION**

The present invention relates to a pharmaceutical combination suitable for the treatment of cancer and other proliferative disorders.

**BACKGROUND TO THE INVENTION**

Initiation, progression, and completion of the mammalian cell cycle are regulated by various cyclin-dependent kinase (CDK) complexes, which are critical for cell growth. These complexes comprise at least a catalytic (the CDK itself) and a regulatory (cyclin) subunit. Some of the more important complexes for cell cycle regulation include cyclin A (CDK1 - also known as cdc2, and CDK2), cyclin B1-B3 (CDK1), cyclin C (CDK8), cyclin D1-D3 (CDK2, CDK4, CDK5, CDK6), cyclin E (CDK2), cyclins K and T (CDK9) and cyclin H (CDK7). Each of these complexes is involved in a particular phase of the cell cycle.

The activity of CDKs is regulated post-translationally, by transitory associations with other proteins, and by alterations of their intracellular localisation. Tumour development is closely associated with genetic alteration and deregulation of CDKs and their regulators, suggesting that inhibitors of CDKs may be useful anti-cancer therapeutics. Indeed, early results suggest that transformed and normal cells differ in their requirement for *e.g.* cyclin A/CDK2 and that it may be possible to develop novel antineoplastic agents devoid of the general host toxicity observed with conventional cytotoxic and cytostatic drugs.

The function of CDKs is to phosphorylate and thus activate or deactivate certain proteins, including *e.g.* retinoblastoma proteins, lamins, histone H1, and components of the mitotic spindle. The catalytic step mediated by CDKs involves a phospho-transfer reaction from ATP to the macromolecular enzyme substrate. Several groups of compounds (reviewed in *e.g.* N. Gray, L. Détivaud, C. Doerig, L. Meijer, Curr. Med. Chem. 1999, 6, 859) have been found to possess anti-proliferative properties by virtue of CDK-specific ATP antagonism.

M.V. Motwani et al. (XP008008541) discloses that **DOCETAXEL AND NAVELBINE INDUCED APOPTOSIS IS ENHANCED BY FLAVOPIRIDOL (FLAVO) IN BREAST CANCER CELLS AND IS SEQUENCE DEPENDENT.**

Roscovitine is the compound 6-benzylanxko-2-[(R)-1-ethyl-2-hydroxyethylamino]-9-isopropylpurine. Roscovitine has been demonstrated to be a potent inhibitor of cyclin dependent kinase enzymes, particularly CDK2. This compound is currently in development as an anti-cancer agent. CDK inhibitors are understood to block passage of cells from the G2/M phase of the cell cycle.

It well established in the art that active pharmaceutical agents can often be given in combination in order to optimise the treatment regime. The present invention therefore seeks to provide a new combination of known pharmaceutical agents that is particularly suitable for the treatment of proliferative disorders, especially cancer. More specifically, the invention centres on the surprising and unexpected effects associated with using certain pharmaceutical agents in combination.

**STATEMENT OF INVENTION**

In a first aspect, the invention provides a combination comprising docetaxel, or a derivative thereof, and roscovitine.

A second aspect provides a pharmaceutical composition comprising a combination according the invention admixed with a pharmaceutically acceptable carrier, diluent or excipient.

A third aspect relates to the use of a combination according the invention in the preparation of a medicament for treating a proliferative disorder

A fourth aspect relates to a pharmaceutical product comprising docetaxel, or a derivative thereof, and roscovitine as a combined preparation for simultaneous, sequential or separate use in therapy.

A fifth aspect relates to a method of treating a proliferative disorder, said method comprising simultaneously, sequentially or separately administering docetaxel, or a derivative thereof, and roscovitine to a subject.

A sixth aspect relates to the use of roscovitine in the preparation of a medicament for the treatment of a proliferative disorder, wherein said treatment comprises simultaneously, sequentially or separately administering docetaxel, or a derivative thereof, and roscovitine to a subject.

A seventh aspect relates to the use of docetaxel, or a derivative thereof, and roscovitine in the preparation of a medicament for treating a proliferative disorder.

An eighth aspect relates to the use of roscovitine in the preparation of a medicament for the treatment of a proliferative disorder, wherein said medicament is for use in combination therapy with docetaxel, or a derivative thereof.

A ninth aspect relates to the use of docetaxel, or a derivative thereof, in the preparation of a medicament for the treatment of a proliferative disorder, wherein said medicament is for use in combination therapy with roscovitine.

## DETAILED DESCRIPTION

The effect of drug combinations is inherently unpredictable and there is often a propensity for one drug to partially or completely inhibit the effects of the other. The present invention is based on the surprising observation that administering docetaxel and roscovitine in combination, either simultaneously, separately or sequentially, does not lead to any adverse interaction between the two agents. The unexpected absence of any such antagonistic interaction is critical for clinical applications.

Preferably, the combination has a synergistic effect, i.e. the combination is synergistic.

Thus, the combination of docetaxel and roscovitine produces an enhanced effect as compared to either drug administered alone. The surprising nature of this observation is in contrast to that expected on the basis of the prior art.

The preferred embodiments as set out below are applicable to all the above-mentioned aspects of the invention.

Docetaxel is an anticancer drug of the taxane family which is prepared by hemisynthesis from the renewable needles of the European yew tree (Taxus baccata). Docetaxel is widely used in the clinic to treat a number of cancers including locally advanced or metastatic breast cancer, locally advanced or metastatic non-small cell lung cancer and hormone refractory prostate cancer.

The mechanism of action is based upon disruption of the microtubular network which plays an essential role in cell division. More recent studies have focused on the use of docetaxel in the first line treatment of non-small cell lung cancer alone or in combination, head and neck cancer, breast cancer, gastric cancer, prostate cancer and ovarian cancer.

Roscovitine is the compound 2-[(1-ethyl-2-hydroxyethyl)amino]-6-benzylamine-9-isopropylpurine, also described as 2-(1-D,L-hydroxymethylpropylamino)-6-benzylamine-9-isopropylpurine. As used herein, the term "roscovitine" encompasses the resolved R and S enantiomers, mixtures thereof, and the racemate thereof.

The *in vitro* activity of roscovitine is as follows:

| Kinase | $IC_{50}$ ($\mu$M) |
|---|---|
| Cdk1/cyclin B | 2.7 |
| Cdk2/cyclin A | 0.7 |
| Cdk2/cyclin E | 0.1 |
| Cdk7/cyclin H | 0.5 |
| Cdk9/cyclin T1 | 0.8 |
| Cdk4/cyclin D1 | 14.2 |
| ERK-2 | 1.2 |
| PKA | >50 |
| PKC | >50 |

The term "proliferative disorder" is used herein in a broad sense to include any disorder that requires control of the cell cycle, for example cardiovascular disorders such as restenosis and cardiomyopathy, auto-immune disorders such as glomerulonephritis and rheumatoid arthritis, dermatological disorders such as psoriasis, anti-inflammatory, antifungal, antiparasitic disorders such as malaria, emphysema and alopecia. In these disorders, the compounds of the present invention may induce apoptosis or maintain stasis within the desired cells as required. Preferably, the proliferative disorder is a cancer or leukaemia, most preferably cancer.

In one preferred embodiment, the cancer is breast cancer, non small cell lung cancer or prostate cancer.

In a particularly preferred embodiment, the invention relates to the use of the combination described hereinbefore in the treatment of a CDK dependent or sensitive disorder. CDK dependent disorders are associated with an above normal level of activity of one or more CDK enzymes. Such disorders are preferably associated with an abnormal level of activity of CDK2 and/or CDK4. A CDK sensitive disorder is a disorder in which an aberration in the CDK level is not the primary cause, but is downstream of the primary metabolic aberration. In such scenarios, CDK2 and/or CDK4 can be said to be part of the sensitive metabolic pathway and CDK inhibitors may therefore be active in treating such disorders. Such disorders are preferably cancer or leukaemic disorders.

As used herein the phrase "preparation of a medicament" includes the use of the components of the invention directly as the medicament in addition to their use in any stage of the preparation of such a medicament.

A further aspect of the invention relates to the use of docetaxel, or a derivative thereof, in the preparation of a medicament for the treatment of a proliferative disorder, wherein said medicament is for use in combination therapy with roscovitine.

As used herein, the term "combination therapy" refers to therapy in which the docetaxel, or derivative thereof, and roscovitine are administered, if not simultaneously, then sequentially within a timeframe that they both are available to act therapeutically within the same time-frame.

As mentioned above, one aspect of the invention relates to a pharmaceutical product comprising roscovitine and docetaxel as a combined preparation for simultaneous, sequential or separate use in therapy.

Roscovitine and docetaxel (or prodrug or derivative thereof) may be administered simultaneously, in combination, sequentially or separately (as part of a dosing regime).

As used herein, "simultaneously" is used to mean that the two agents are administered concurrently, whereas the term "in combination" is used to mean they are administered, if not simultaneously, then "sequentially" within a timeframe that they both are available to act therapeutically within the same time-frame. Thus, administration "sequentially" may permit one agent to be administered within 5 minutes, 10 minutes or a matter of hours after the other provided the circulatory half-life of the first administered agent is such that they are both concurrently present in therapeutically effective amounts. The time delay between administration of the components will vary depending on the exact nature of the components, the interaction therebetween, and their respective half-lives.

In contrast to "in combination" or "sequentially", "separately" is used herein to mean that the gap between administering one agent and the other is significant i.e. the first administered agent may no longer be present in the bloodstream in a therapeutically effective amount when the second agent is administered.

In one preferred embodiment of the invention, roscovitine is administered simultaneously, with the docetaxel.

In another preferred embodiment, roscovitine is administered sequentially with the docetaxel.

In one preferred embodiment of the invention, roscovitine is administered sequentially or separately prior to the docetaxel. Preferably, roscovitine is administered at least 4 hours before the docetaxel, and more preferably at least 72 hours before the docetaxel.

In another preferred embodiment, roscovitine is administered both prior to and subsequent to administration of the docetaxel.

Thus, in one especially preferred embodiment, docetaxel is administered as single i.v. injection after 2 days of therapy with roscovitine, followed by another 2 days with roscovitine. More preferably, the cycle is repeated after 14 days.

In a particularly preferred embodiment, the docetaxel is administered sequentially or separately prior to roscovitine. Preferably, the docetaxel is administered at least 1 hour before roscovitine, and more preferably at least 24 hours before the CDK inhibitor.

In one particularly preferred embodiment, the docetaxel is administered as a single i.v. injection 1 day before roscovitine is given for 4 consecutive days. More preferably, the same cycle is repeated after 2 weeks.

In one preferred embodiment, roscovitine and docetaxel are each administered in a therapeutically effective amount with respect to the individual components; in other words, roscovitine and docetaxel are administered in amounts that would be therapeutically effective even if the components were administered other than in combination.

In another preferred embodiment, roscovitine and docetaxel are each administered in a sub-therapeutic amount with respect to the individual components; in other words, roscovitine and docetaxel are administered in amounts that would be therapeutically ineffective if the components were administered other than in combination.

Preferably, the docetaxel and roscovitine inhibitor interact in a synergistic manner. As used herein, the term "synergistic" means that docetaxel and roscovitine produce a greater effect when used in combination than would be expected from adding the individual effects of the two components. Advantageously, a synergistic interaction may allow for lower doses of each component to be administered to a patient, thereby decreasing the toxicity of chemotherapy, whilst producing and/or maintaining the same therapeutic effect. Thus, in a particularly preferred embodiment, each component can be administered in a sub-therapeutic amount.

Evidence in support of a synergistic interaction is detailed in the accompanying

examples.

## SALTS/ESTERS

The agents of the present invention can be present as salts or esters, in particular pharmaceutically acceptable salts or esters.

Pharmaceutically acceptable salts of the agents of the invention include suitable acid addition or base salts thereof. A review of suitable pharmaceutical salts may be found in Berge et al, J Pharm Sci, 66, 1-19 (1977). Salts are formed, for example with strong inorganic acids such as mineral acids, e.g. sulphuric acid, phosphoric acid or hydrohalic acids; with strong organic carboxylic acids, such as alkanecarboxylic acids of 1 to 4 carbon atoms which are unsubstituted or substituted (e.g., by halogen), such as acetic acid; with saturated or unsaturated dicarboxylic acids, for example oxalic, malonic, succinic, maleic, fumaric, phthalic or tetraphthalic; with hydroxycarboxylic acids, for example ascorbic, glycolic, lactic, malic, tartaric or citric acid; with aminoacids, for example aspartic or glutamic acid; with benzoic acid; or with organic sulfonic acids, such as ($C_1$-$C_4$)-alkyl- or aryl-sulfonic acids which are unsubstituted or substituted (for example, by a halogen) such as methane- or p-toluene sulfonic acid.

Esters are formed either using organic acids or alcohols/hydroxides, depending on the functional group being esterified. Organic acids include carboxylic acids, such as alkanecarboxylic acids of 1 to 12 carbon atoms which are unsubstituted or substituted (e.g., by halogen), such as acetic acid; with saturated or unsaturated dicarboxylic acid, for example oxalic, malonic, succinic, maleic, fumaric, phthalic or tetraphthalic; with hydroxycarboxylic acids, for example ascorbic, glycolic, lactic, malic, tartaric or citric acid; with aminoacids, for example aspartic or glutamic acid; with benzoic acid; or with organic sulfonic acids, such as ($C_1$-$C_4$)-alkyl- or aryl-sulfonic acids which are unsubstituted or substituted (for example, by a halogen) such as methane- or p-toluene sulfonic acid. Suitable hydroxides include inorganic hydroxides, such as sodium hydroxide, potassium hydroxide, calcium hydroxide, aluminium hydroxide. Alcohols include alkanealcohols of 1-12 carbon atoms which may be unsubstituted or substituted, e.g. by a halogen).

## ENANTIOMERS/TAUTOMERS

The invention also includes where appropriate all enantiomers and tautomers of the agents. The man skilled in the art will recognise compounds that possess an optical properties (one or more chiral carbon atoms) or tautomeric characteristics. The corresponding enantiomers and/or tautomers may be isolated/prepared by methods known in the art.

## STEREO AND GEOMETRIC ISOMERS

Some of the agents of the invention may exist as stereoisomers and/or geometric isomers - e.g. they may possess one or more asymmetric and/or geometric centres and so may exist in two or more stereoisomeric and/or geometric forms. The present invention contemplates the use of all the individual stereoisomers and geometric isomers of those inhibitor agents, and mixtures thereof. The terms used in the claims encompass these forms, provided said forms retain the appropriate functional activity (though not necessarily to the same degree).

The present invention also includes all suitable isotopic variations of the agent or pharmaceutically acceptable salts thereof. An isotopic variation of an agent of the present invention or a pharmaceutically acceptable salt thereof is defined as one in which at least one atom is replaced by an atom having the same atomic number but an atomic mass different from the atomic mass usually found in nature. Examples of isotopes that can be incorporated into the agent and pharmaceutically acceptable salts thereof include isotopes of hydrogen, carbon, nitrogen, oxygen, phosphorus, sulphur, fluorine and chlorine such as $^2$H, $^3$H, $^{13}$C, $^{14}$C, $^{15}$N, $^{17}$O, $^{18}$O, $^{31}$P, $^{32}$P, $^{35}$S, $^{18}$F and $^{36}$Cl, respectively. Certain isotopic variations of the agent and pharmaceutically acceptable salts thereof, for example, those in which a radioactive isotope such as $^3$H or $^{14}$C is incorporated, are useful in drug and/or substrate tissue distribution studies. Tritiated, i.e., $^3$H, and carbon-14, i.e., $^{14}$C, isotopes are particularly preferred for their ease of preparation and detectability. Further, substitution with isotopes such as deuterium, i.e., $^2$H, may afford certain therapeutic advantages resulting from greater metabolic stability, for example, increased *in vivo* half-life or reduced dosage requirements and hence may be preferred in some circumstances. Isotopic variations of the agent of the present invention and pharmaceutically acceptable salts thereof of this invention can generally be prepared by conventional procedures using appropriate isotopic variations of suitable reagents.

## SOLVATES

The present invention also includes solvate forms of the agents of the present invention. The terms used in the claims encompass these forms.

## POLYMORPHS

The invention furthermore relates to agents of the present invention in their various crystalline forms, polymorphic forms and (an)hydrous forms. It is well established within the pharmaceutical industry that chemical compounds may be isolated in any of such forms by slightly varying the method of purification and or isolation form the solvents used in the synthetic preparation of such compounds.

## CHEMICAL DERIVATIVE

The term "derivative" as used herein includes chemical modification of one or more of the agents of the invention. Illustrative of such chemical modifications would be replacement of hydrogen by a halo group, an alkyl group, an acyl group or an amino group.

## ADMINISTRATION

The pharmaceutical compositions of the present invention may be adapted for oral, rectal, vaginal, parenteral, intramuscular, intraperitoneal, intraarterial, intrathecal, intrabronchial, subcutaneous, intradermal, intravenous, nasal, buccal or sublingual routes of administration.

For oral administration, particular use is made of compressed tablets, pills, tablets, gellules, drops, and capsules. Preferably, these compositions contain from 1 to 2000 mg and more preferably from 50-1000 mg, of active ingredient per dose.

Other forms of administration comprise solutions or emulsions which may be injected intravenously, intraarterially, intrathecally, subcutaneously, intradermally, intraperitoneally or intramuscularly, and which are prepared from sterile or sterilisable solutions. The pharmaceutical compositions of the present invention may also be in form of suppositories, pessaries, suspensions, emulsions, lotions, ointments, creams, gels, sprays, solutions or dusting powders.

An alternative means of transdermal administration is by use of a skin patch. For example, the active ingredient can be incorporated into a cream consisting of an aqueous emulsion of polyethylene glycols or liquid paraffin. The active ingredient can also be incorporated, at a concentration of between 1 and 10% by weight, into an ointment consisting of a white wax or white soft paraffin base together with such stabilisers and preservatives as may be required.

Injectable forms may contain between 10 - 1000 mg, preferably between 10 - 500 mg, of active ingredient per dose.

Compositions may be formulated in unit dosage form, i.e., in the form of discrete portions containing a unit dose, or a multiple or sub-unit of a unit dose.

In a particularly preferred embodiment, the combination or pharmaceutical composition of the invention is administered intravenously.

## DOSAGE

A person of ordinary skill in the art can easily determine an appropriate dose of one of the instant compositions to administer to a subject without undue experimentation. Typically, a physician will determine the actual dosage which will be most suitable for an individual patient and it will depend on a variety of factors including the activity of the specific compound employed, the metabolic stability and length of action of that compound, the age, body weight, general health, sex, diet, mode and time of administration, rate of excretion, drug combination, the severity of the particular condition, and the individual undergoing therapy. The dosages disclosed herein are exemplary of the average case. There can of course be individual instances where higher or lower dosage ranges are merited, and such are within the scope of this invention.

Depending upon the need, the agent may be administered at a dose of from 0.1 to 30 mg/kg body weight, such as from 0.1 to 10 mg/kg, more preferably from 2 to 20 mg/kg body weight.

By way of guidance, docetaxel is typically administered in accordance to a physicians direction at dosages between 60 and 100 mg/m$_2$ body surface intravenously over one hour every 21 days or at dosages between 20 and 40 mg/m$^2$ intravenously over one hour every week for up to 6 month. Dosages and frequency of application are typically adapted, to the general medical condition of the patient and to the severity of the adverse effects like hypersensitivity reactions and to those adverse effects caused to the hematopoietic and to the nervous system.

Roscovitine is typically administered from about 0.05 to about 5g/day, preferably from about 0.4 to about 3 g/day. Roscovitine is preferably administered orally in tablets or capsules. The total daily dose of roscovitine can be administered as a single dose or divided into separate dosages administered two, three or four time a day.

Preferably, roscovitine is administered as an orally or intravenously at a dosage of from 0.4 to 3 g/day. Docetaxel is then administered in the manner deemed most suitable at an appropriate dosage as discussed above. Preferably, the docetaxel is administered at least 24 hours after the administration of roscovitine.

The present invention is further described by way of example and with reference to the following Figures wherein:

Figure 1 shows the effect of 24 hour pre-exposure with roscovitine followed by 24 hour docetaxel exposure.

Figure 2 shows the effect of roscovitine (CYC-202) and docetaxel (taxotere) administration on tumour size in a MAXF 857 primary breast cancer xenograft. In more detail, Figure 2 shows median relative tumour volume against time (days after randomization) for treatment with roscovitine alone, docetaxel alone and a roscovitine/docetaxel combination versus a control.

Figures 3A and 3B shows the effect of docetaxel/roscovitine scheduling in a lung cancer xenograft (LXFL H460).

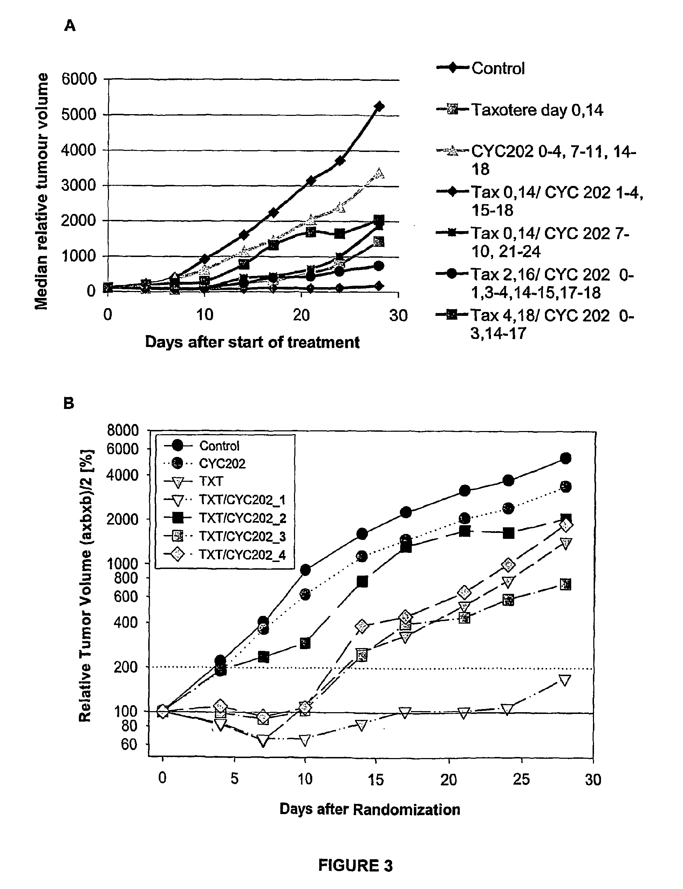

In more detail, Figure 3 shows median relative tumour volume against time (days after start of treatment) for docetaxel alone, roscovitine alone and various roscovitine/docetaxel schedules.

## EXAMPLES

The growth inhibitory activity of roscovitine was measured alone and in combination with docetaxel against MDA-435 breast tumour cell line and PCM-3 prostate cell line using a monolayer assay and a tumour stem cell assay.

Methods and Materials

Compound

Stock solutions of CDK inhibitor (for example roscovitine) were prepared in DMSO and aliquots stored at -20°C. Final dilutions were prepared in culture medium (Iscove's Modified Dulbecco's Medium; Life Technologies, Karlsruhe) immediately prior to use.

Clonogenic Assay

Preparation of Single cell suspensions from human tumor xenografts

Solid human tumor xenografts growing subcutaneously in serial passages in thymus aplastic nude mice (NMRI, Naval Medical Research Institute, USA, nu/nu strain, obtained from our own breeding facility) were removed under sterile conditions, mechanically disaggregated and subsequently incubated with an enzyme cocktail consisting of collagenase (41 U/ml, Sigma), DNAse I (125 U/ml, Roche), hyaluronidase (100 U/ml, Sigma) and dispase II (1.0 U/ml, Roche) in RPMI 1640-Medium (Life Technologies) at 37°C for 30 minutes. Cells were passed through sieves of 200 $\mu$m and 50 $\mu$m mesh size and washed twice with sterile PBS-buffer (Life Technologies). The percentage of viable cells was determined in a Neubauer-hemocytometer using trypan blue exclusion.

Culture methods

The clonogenic assay was performed in a 24-well format according to a modified two-layer soft agar assay introduced by Hamburger & Salmon [Alley, M.C., Uhi, C.B. & M.M. Lieber, 1982]. Improved detection of drug cytotoxicity in the soft agar colony formation assay through use of a metabolizable tetrazolium salt. Life Sci. 31: 3071-3078]. The bottom layer consisted of 0.2 ml/well of Iscove's Modified Dulbecco's Medium (supplemented with 20% (v/v) fetal calf serum and 0.01% (v/v) gentamicin) and 0.75% (w/v) agar. $4 \cdot 10^4$ to $8 \cdot 10^4$ cells were added to 0.2 ml of the same culture medium supplemented with 0.4% (w/v) agar and plated in 24-multiwell dishes onto the bottom layer. Cytostatic drugs were applied by continuous exposure (drug overlay) in 0.2 ml culture medium. Every dish included six control wells containing the vehicle and drug treated groups in triplicate at 6 concentrations. Cultures were incubated at 37°C and 7.5% $CO_2$ in a humidified atmosphere for 8 - 20 days and monitored closely for colony growth using an inverted microscope. Within this period, *in vitro* tumor growth led to the formation of colonies with a diameter of > 50$\mu$m. At the time of maximum colony formation, counts were performed with an automatic image analysis system (OMNICON FAS IV, Biosys GmbH). 24 hours prior to evaluation, vital colonies were stained with a sterile aqueous solution of 2-(4-iodophenyl)-3-(4-nitrophenyl)-5-phenyltetrazolium chloride (1 mg/ml, 100 $\mu$l/well) [i].

An assay was considered fully evaluable, if the following quality control criteria were fulfilled:

- Mean number of colonies in the control group wells of 24-multiwell plates $\geq$ 20 colonies with a colony diameter of > 50 $\mu$m
- The positive reference compound 5-fluorouracil (5_FU) (at the toxic dose of 1000 $\mu$g/ml) must effect a colony survival of < 20% of the controls
- Initial plate counts on day 0 or 2 < 20% of the final control group count
- Coefficient of variation in the control group $\leq$ 50%

Data evaluation

Drug effects were expressed in terms of the percentage of survival, obtained by comparison of the mean number of colonies in the treated plates with the mean colony count of the untreated controls (relative colony count expressed by the test-versus-control-group value, T/C-value [%]):

$$\frac{T}{C} = \frac{\text{colony count}_{treated\ group}}{\text{colony count}_{control\ group}} \cdot 100\ [\%].$$

IC50- and IC70-values, being the drug concentration necessary to inhibit colony formation by 50% (T/C = 50%) and 70% (T/C = 30%) respectively, were determined by plotting compound concentration versus relative colony count. Mean IC50- and IC70-values were calculated according to the formula

$$\text{mean IC}_{50,70} = 10^{\left(\frac{\sum_{x=1}^{n} \lg(IC_{50,70})}{n}\right)}$$

with x the specific tumor model, and n the total number of tumor models studied. If an IC50- or IC70-value could not be determined within the examined dose range, the lowest or highest concentration studied was used for the calculation.

In the mean graph analysis (IC-plot) the distribution of IC70-values obtained for a test compound in the individual tumor types is given in relation to the mean IC70-value, obtained for all tumors tested. The individual IC70-values are expressed as bars in a logarithmically scaled axis. Bars to the left demonstrate IC70-values lower than the mean value (indicating more sensitive tumor models), bars to the right demonstrate higher values (indicating rather resistant tumor models). The IC-plot therefore represents a fingerprint of the antiproliferative profile of a compound.

Test procedure: Combination of roscovitine with standard agents

Cell lines

The characteristics of the 6 human tumor cell lines are shown in Table 1 below.

Table 1: Cell Lines used for Testing Roscovitine in Combination with standard agents

| Tumor Type Formation | Cell Line | Histology in nude mice | Doubling Time [h] | Tumor in vivo |
|---|---|---|---|---|
| Colon | DLD1 | adeno ca | nd | yes |
| | HT29 | pd adeno ca | 23 | yes |
| Lung, NSC | LXFA 629L | adeno carcinoma | 31 | yes |
| Prostate | 22RV1 | nd | 40 | yes |
| | DU145 | adeno ca | nd | yes |
| | PC3M | pd adeno ca | nd | yes |

ud = undifferentiated, pd = poorly differentiated, md = moderately differentiated, wd = well differentiated, mm = malignant melanoma; ND = not determined

The lung carcinoma cell line LXFA 629L was established from a human tumor xenograft as described by Roth et al. 1999 [Roth T, Burger AM, Dengler W, Willmann H, Fiebig HH. Human tumor cell lines demonstrating the characteristics of patient tumors as useful models for anticancer drug screening. In: Fiebig HH, Burger AM (eds). Relevance of Tumor Models for Anticancer Drug Development. Contrib. Oncol. 1999, 54: 145-156]. The origin of the donor xenograft was described by Fiebig et al. 1992 [Fiebig HH, Dengler WA, Roth T. Human tumor xenografts: Predictivity, characterization, and discovery of new anticancer agents. In: Fiebig HH, Burger AM (eds). Relevance of Tumor Models for Anticancer Drug Development. Contrib. Oncol. 1999, 54: 29 - 50].

The cell lines DLD1 and HT29 (colon), as well as the prostate carcinoma DU145 and PC3M were obtained from US-NCI (National Cancer Institute, USA).

The prostate carcinoma 22RV1 was purchased from the American Type Culture Collection (ATCC).

Cells were routinely passaged once or twice weekly. They were maintained no longer than 20 passages in culture. All cells were grown at 37°C in a humidified atmosphere (95% air, 5% $CO_2$) in RPMI 1640 medium (Invitrogen, Karlsruhe, Germany) supplemented with 10% fetal calf serum (Sigma, Deisenhofen, Germany) and 0.1% gentamicin (Invitrogen).

Cell proliferation assay

A modified propidium iodide assay was used to assess the effects of roscovitine on the growth of the human tumor cell lines [Dengler WA, Schulte J, Berger DP et al. (1995). Development of a propidium iodide fluorescence assay for proliferation and cytotoxicity assay. Anti-Cancer Drugs 1995, 6:522-532]. Briefly, cells are harvested from exponential phase cultures by trypsination, counted and plated in 96 well flat-bottomed microtiter plates at a cell density dependent on the cell line (5 - 12.000 viable cells/well). After 24 h recovery to allow the cells to resume exponential growth, 20 $\mu$l of culture medium (3 control wells per plate) or culture medium containing various concentrations of test article no. 1 (standard agent) was added to the wells. Each concentration was plated in triplicate. On each plate test article no. 1 is applied in five concentrations 4 times in 4 quarters of the microtiter plate. Quarter 1 was for the test article no.1 alone, in quarters 2-4 the test article no. 2 (roscovitine) was applied at three different time points, respectively. Following 4 days of continuous test article exposure, cell culture medium with or without drug was replaced by 200 $\mu$l of an aqueous propidium iodide (PI) solution (7 $\mu$g/ml). Since PI only passes through leaky or lysed cell membranes, DNA of dead cells could be stained and measured, while living cells were not be stained. To measure the proportion of living cells, cells were permeabilized by freezing the plates, resulting in death of all cells. After thawing of the plates fluorescence was then measured using the Cytofluor 4000 microplate reader (excitation 530 nm, emission 620 nm), giving a direct relationship to the total cell number. Growth inhibition was expressed as treated/control x 100 (%T/C) and $IC_{50}$, $IC_{70}$ and $IC_{90}$ values for each combination were determined by plotting compound concentration versus cell viability.

MTT Assay

The MTT assay was used to evaluate the effects of roscovitine with and without docetaxel. The MTT assay is a spectrophotometric assay based on the ability of viable cells to convert MTT to formazan. Cell concentrations were estimated by measuring absorbance at test wavelength of 570 nm and a reference wavelength of 630 nm. An automated procedure was utilized to determine the $IC_{50}$ value (concentration of drug which inhibits cell growth by 50% of the control) of all agents used in these studies. Cell lines were selected with specific possibilities in mind for future clinical trial designs. Initially, roscovitine and docetaxel were tested separately over a range of concentrations. After the initial $IC_{50}$ analysis was complete, the combinations were then tested. For the combination studies, the concentration (expressed as a percent of the individual agent's $IC_{50}$) schema used to characterise the type of interaction is shown below:

Drug Concentration (Expressed as a percent of the $IC_{50}$)

| Roscovitine | Docetaxel |
| --- | --- |
| 100 | 0 |
| 75 | 25 |
| 60 | 40 |
| 50 | 50 |
| 40 | 60 |
| 25 | 75 |
| 0 | 100 |
| 0 | 0 |

Statistical Analysis of Combination Studies

To interpret the combination curves, statistical comparisons were made with each test combination (75:25 roscovitine/docetaxel) and the endpoints (100:0-roscovitine and 0:100-docetaxel). A statistically significant observation requires that a difference exists between the combination (roscovitine and docetaxel) absorbance value and both endpoint values (roscovitine and docetaxel alone) [Greco et al, The search for synergy; A critical review from a response surface perspective. Pharmacol; Review 47:331-385, 1995; Laska et al, Simple designs and model-free tests for synergy; Biometrics 50:834-841,1994]. If the majority of ($\geq$3 of 5) of the values are statistically above or below the line (endpoints) then antagonism or synergy is described, respectively. Otherwise, the pattern is more consistent with an additive interaction.

Results

Roscovitine exposure followed by Docetaxel

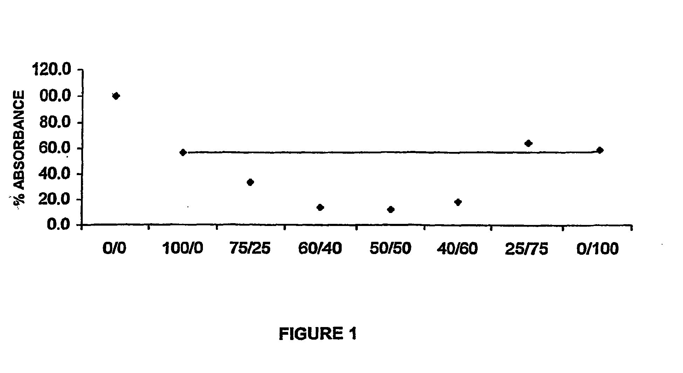

In these studies, breast tumor cells (MDA-435) were pre-exposed for 24 hours to roscovitine followed by 24 hour exposure to docetaxel (Tables 2 and 3, Figure 1). This sequence of exposure to both agents resulted in a pattern suggestive of a synergistic interaction between these agents.

**Table 2**

| %IC$_{50}$ | Concentration | | Tax. (ng/ml) | Well 1 | Well 2 | Well 3 | Mean | % Abs | % Response |
|---|---|---|---|---|---|---|---|---|---|
| | ROSCOVITINE (μg/ml) | | | | | | | | |
| 0/0 | 0 | | 0 | 1.321 | 1.410 | 1.305 | 1.345 | 100.0 | |
| 100/0 | 15 | | 0 | 0.775 | 0.740 | 0.775 | 0.763 | 0 | 43.3 |
| 75/25 | 11.25 | | 0.45 | 0.439 | 0.425 | 0.492 | 0.452 | 56.7 | 66.4 |
| 60/40 | 9 | | 0.72 | 0.159 | 0.191 | 0.216 | 0.189 | 33.6 | 86.0 |
| 50/50 | 7.5 | | 0.9 | 0.165 | 0.154 | 0.174 | 0.164 | 14.0 | 87.8 |
| 40/60 | 6 | | 1.08 | 0.268 | 0.190 | 0.302 | 0.253 | 12.2 | 81.2 |
| 25/75 | 3.75 | | 1.35 | 0.875 | 0.782 | 0.961 | 0.873 | 18.8 | 35.1 |
| 0/100 | 0 | | 1.8 | 0.873 | 0.771 | 0.749 | 0.798 | 64.9 59.3 | 40.7 |

**Table 3**

| | p-values 100/0 | p-values 0/100 |
|---|---|---|
| 100/0 | | |
| 75/25 | 0.00018771624 | 0.00133536059 |
| 60/40 | 0.00000909306 | 0.00012676295 |
| 50/50 | 0.00000133568 | 0.00008105605 |
| 40/60 | 0.00013113980 | 0.00042261143 |
| 25/75 | 0.10803231506 | 0.30804956660 |
| 0/100 | | |

Human Tumour Xenograft Studies

The role of roscovitine in combination with docetaxel was investigated in human tumor xenografts *in vivo*. The NCI derived human lung cancer H460 was grown as xenograft subcutaneously in immune deficient nude mice. Special focus was put on different schedules combining roscovitine and docetaxel.

Animal Information

| Specific Information | |
|---|---|
| Mouse strain: | NMRI nu/nu |
| Animals supplied by: | Institute of Experimental Oncology, Oncotest GmbH, Freiburg, Germany |
| Gender of mice: | females and males |
| Median body weight at randomization: | female: 28-32 gram male: 30-36 gram |
| Approximate age at randomization: | 6 - 9 weeks |

Animal Health and Acclimatization Period

All experiments were conducted according to the guidelines of the German Animal Health and Welfare Act (Tierschutzgesetz).

Animal health was examined at the day before tumour implantation and before randomization to ensure that only animals of good health were selected to enter testing procedures.

Grouping and Randomization of Animals

The animals were arbitrarily numbered during the tumour implantation using ear clips. The day before the first administration of a compound tumour bearing animals were stratified into several groups according to tumour volume. Only animals with appropriate tumour volumes were selected and randomly distributed to treatment and control groups. Randomization was performed using "Lindner's Randomization Tables". Each group consisted of 6-9 mice. The tumour volumes at the start of treatment were 86 mm$^3$ in PC3M, 103 mm$^3$ in DU145, 186 mm$^3$ in MAXF 857 and 75 mm$^3$ in LXFL H460.

Animal Identification

An individual ear tag number identified each mouse. At the beginning of the study, each cage was labeled with a record card, indicating the experimental number, date of tumour implantation, date of randomization, tumour type, tumour number, mouse strain, gender, and individual mouse number. After randomization group identity, test compound, dosage, schedule, and route of administration were added.

Housing Conditions

Husbandry

The animals were housed in Macrolon$^M$ type III cages with filter hoods (maximum 8 mice/cage) in laminar flow rackets. The cages were located in a separate room at Oncotest. The cages were sterilized at 121°C before use and changed twice a week. Room temperature was maintained at $24\pm1$°C and relative humidity at $50\pm10$%. The animals were kept under a natural daylight cycle.

Diet and Water Supply

The animals were fed Altromin Extrudat 1439 Rat/Mouse diet. The diet was purchased from Altromin GmbH (Lage, Germany).

Water was sterilized at 121°C for 30 minutes. After sterilization 0.9 g/l potassium sorbate was added, the pH was adjusted to 2 with 1N HCl. Water consumption was visually monitored daily, the bottles were changed two times a week. Food and water were provided *ad libitum*.

Bedding

The animal bedding S 8/15 produced by Rettenmaier & Söhne Faserstoffwerke (Ellwangen-Holzmühle, Germany) was used. The bedding was sterilized at 130°C for 15 minutes and renewed twice a week. The bedding was delivered by E. Müller (Forchheim, Germany).

The producer analyzes the dust free bedding every three month with respect to biological/fungal contamination and content of phosphate esters, arsenic, cadmium, lead and mercury. These analyses are carried out at the Agriculture Analyses and Research Institute, Ministry of Agriculture, Kiel, Germany. The quality certificates are deposited at Rettenmaier & Söhne Faserstoffwerke (Ellwangen-Holzmühle, Germany).

Tumour Information

Characterization of the Tumour Model

The human lung tumour cell line H460 was obtained from the US National Cancer Institute. This tumour cell line was established in vivo by subcutaneous injection into nude mice and tumours propagated until stable growth behavior was obtained.

MAXF 857 was established from patients treated at the department of internal medicine, University of Freiburg.

They were established by implanting patient tissue into the nude mouse and tumours propagated until stable tumour growth behavior was observed. Master stocks from early passages were frozen in liquid nitrogen. A particular master stock batch is only used for about 10 further passages.

Implantation of the Human Tumour Xenografts

The fragments were obtained from in serial passage in nude mice. After removal of the tumour from the donor mice, the tumour was cut into fragments (1-2 mm diameter) and placed in RPMI 1640 culture medium until subcutaneous implantation in the mice.

The mice were anaesthetized by inhalation of isoflurane. A small incision was made in the skin of the back. The tumour fragments (two fragments per mouse) were transplanted with tweezers. The mice were monitored daily.

Test Substances

Roscovitine (supplied by Cyclacel Ltd) was stored at 4°C in dark. The substance was dissolved in a buffer containing 50 mM HCl and 0.05% Tween 80. Solutions were prepared fresh daily. Reference compounds were prepared as follows: Docetaxol (Taxotere®, Aventis) was used as clinical formulation.

Treatment Procedure

Route of Administration

Roscovitine and the vehicle (50 mM HCl and 0.05% Tween 80) were administered orally. Taxotere was administered iv.

Drug Dosage and Treatment Regimen

The test compound roscovitine was administered at doses of 300 and 150 mg/kg/day over at least three days. Second treatment cycles were performed one to three weeks after the first cycle. The daily dose was split and administered at hour 0 and 6. Taxotere was administered at optimal dose (20 mg/kg) and scheduling. The standard anticancer agents were administered 15 min. before application of roscovitine when given on the same day.
Control mice received vehicle (10 ml/kg/day) using the administration schedule of roscovitine.
The MTD was defined as the allowance of a median body weight loss of approximately 15% of the initial weight within 2 weeks after the last drug administration and 1 drug-related death out of 3 animals.

Observations

Mortality

Mortality checks were conducted daily. Lethality was considered to be drug related occurring until 7 days after last therapy.

Body Weight

Body weight of the mice was determined twice a week i.e. at the same day when the tumours were measured.
The body weight of a treatment group was expressed at the absolute and relative median body weight of all animals of the group. The relative body weight of each individual animal was calculated according to the formula:

$$\text{Relative Body Weight} = \frac{\text{Body Weight day}_x}{\text{Body Weight day}_0} \times 100$$

Tumour Volume

The tumour volume was determined by two-dimensional measurement with a caliper at the day of randomization (day 0) and than twice weekly. The tumour volume was calculated according to the formula: $(a \times b^2) \times 0.5$ a and b represent two perpendicular tumour diameters: a, larger, b, smaller diameter.

Study Endpoint

All mice of a treatment group had to be sacrificed when the tumours reached mean diameters of approximately 16 mm (2.0 gram) according to the guidelines to conduct animal experiments. Test groups were finished when the initial tumour volume of about 100 mm$^3$ had increased at least 4 fold.

Antitumour Activity

The antitumour effect was evaluated following maximal tumour inhibition versus the control group. Data evaluation was performed using specifically designed software.

Relative Tumour Volume

Relative tumour volumes (RTV) were calculated for each individual tumour by dividing the tumour volume on day X ($V_x$) by the tumour volume on day 0 ($V_0$) multiplied by 100%. The tumour volume of a treatment group was expressed as the median RTV of all mice of the group. Median RTV values were used for drawing growth curves and treatment evaluation.

Tumour Doubling Time

Tumour doubling time (Td) of test and control groups is defined, as the time (days) required in days to reach a median RTV of 200%.

Tumour Inhibition. Test/Control Value in %

Optimal growth inhibition at a particular day within the experimental period was calculated from the median RTV values of the test versus control groups multiplied by 100 (T/C in %).
The relative tumour volume $T_x/T_0$ is defined as follow:

$$T_x/T_0 = \frac{\text{Median relative tumour volume of the test group day}_x}{\text{Median relative tumour volume of the test group day}_0} \times 100$$

Absolute Growth Delay

Absolute growth delay is defined as the difference in days of Td (test) - Td (control).

Efficacy Criteria

The minimum (or optimum) T/C value recorded for a particular test group represents the maximum antitumour activity for the respective treatment.
T/C % and $T_x/T_0$ were used for activity rating of the levels of efficacy, which were defined as follows:

| - | inactive | T/C >=50% |
|------|-------------------|----------------|
| + | moderate activity | T/C 25%-<50% |
| ++ | high activity | T/C 10%-<25% |
| +++ | very high activity | T/C 5%-<10% |
| ++++ | Complete remission | T/C <5% |

Results of Human Tumour Xenograft Studies

Experiment 1

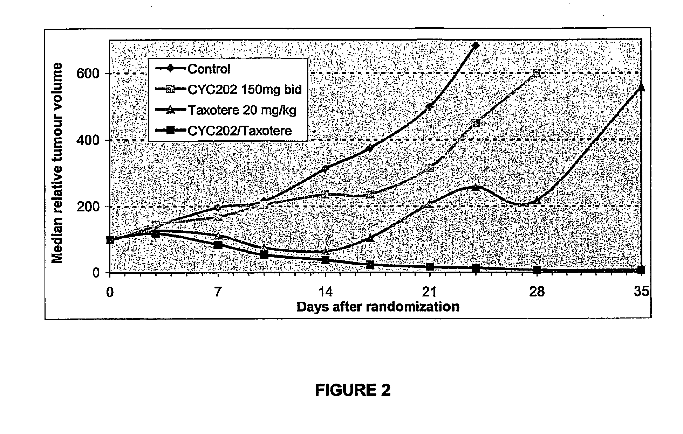

The effect of roscovitine (CYC-202) and docetaxel (taxotere) administration on tumour size in a primary breast cancer xenograft (MAXF 857) is shown in Figure 2. The MAXF 857 xenograft mice were treated with taxotere as a single agent on day 2 after randomization, or treated with roscovitine as a single agent on days 0-3 and 7-9 after randomization, or the combination of the two treatments. Combination of roscovitine with taxotere showed a pronounced synergism. The combination was markedly more active (optimal T/C of 2%) than the monotherapy (optimal T/C of 63% and 21%). This effect was statistically significant with p< 0,001 (U-Test by Mann-Whitney-Wilcox on, day 24).

Experiment 2

Taxotere/roscovitine scheduling in a lung cancer xenograft (LXFL H460) is shown in Figures 3A and 3B, and Table 4 below.

**Table 4**

| Combination schedule | Days After Randomization (0–24) |
| --- | --- |
| control | v v v v v … v v v v v … v v v v v |
| CYC202 | (schedule) |
| TXT | (schedule) |
| 1  TXT/CYC202 | (schedule) |
| 2  TXT/CYC202 | (schedule) |
| 3  TXT/CYC202 | (schedule) |
| 4  TXT/CYC202 | (schedule) |

v, vehicle; CYC202 given bd (150 mg/kg/injection) resulting in 300 mg/kg/d, po; docetaxel given at 20 mg/kg, iv

Toxicities and T/C values are shown below in Table 5.

**Table 5**

| Combination | Therapy | Dose level [mg/kg/d] | Schedule [day] | Lethality n (day)* | Median BWC % (day 21)** | T/C [%] day 28 | T/C opt [%] / day opt |
| --- | --- | --- | --- | --- | --- | --- | --- |
|  | Control |  | bd 0-4,7-11,14-18 | 0/10 | + 6.2 | 100 | 100 / - |
|  | CYC202 | 300 | bd 0-4,7-11,14-18 | 0/10 | + 7.1 | 64.3 | 64.3 / 28 |
|  | TXT | 20 | 0,14 | 0/10 | - 4.2 | 27.3 | 12.2 / 10 |
| 1 | TXT/ CYC202 | 20/ 300 | 0,14/ bd 1-4,15-18 | 0/10 | - 7.1 | 3.3 | 2.9 / 24 |
| 2 | TXT/ CYC202 | 20/ 300 | 4,18/ bd 0-3,14-17 | 0/10 | - 5.2 | 38.9 | 32.2 / 10 |
| 3 | TXT/ CYC202 | 20/ 300 | 2,16/ bd 0-1,3-4,14-15,17-18 | 0/10 | - 11.5 | 14.1 | 11.3 / 10 |
| 4 | TXT/ CYC202 | 20/ 300 | 0,14/ bd 7-10,21-24 | 0/10 | - 11.0 | 35.6 | 11.8 / 10 |

\* Days on which lethality occured
\** Body weight change one week after last therapy of docetaxel

Whereas roscovitine as single agent was shown to exert slight antitumour efficacy (300 mg/kg over 4 days bid, T/C = 64%), the compound at the same dosage increases the efficacy of docetaxel (20 mg/kg, two single injections at intervals of 2 weeks, T/C = 27%) in a schedule-dependent manner.

Best effects (and a marked synergistic effect) were obtained when docetaxel was given as a single i.v. injection one day before roscovitine given for 4 consecutive days, and the same cycle repeated after 2 weeks (Schedule 1; T/C = 3%). Tumor growth delay of this combination in relation to the vehicle treated control was >32 days compared to 9 days with docetaxel in single therapy. This schedule also led to a long lasting antitumour effect. Tumour doubling time was ~28 days versus 3 days in the control.

A slight benefit was observed when docetaxel was given as single injection after 2 days of therapy with roscovitine, followed by another 2 days with roscovitine, the cycle repeated after 14 days (Schedule 3; T/C =14%).

The schedule with docetaxel given one week before 4 days of roscovitine treatment, and the same cycle given 2 weeks later, showed no synergism (Schedule 4; T/C = 36%).

Least benefit was obtained in giving a single injection of docetaxel after having given roscovitine for 4 consecutive days (2 cycles with a 10 day interval) (Schedule 2; T/C = 40%). During the study there was no pronounced toxicity.

In the combination therapies, body weight loss was slightly more pronounced than in single therapy (up to 12% of the median initial body weight), but was within the acceptable range. The schedule dependency has therefore proven to be very critical, with best results obtained when docetaxel was given shortly before a cycle of roscovitine, i.e. one day before roscovitine.

By way of summary, the results demonstrate that the administration of docetaxel in combination with roscovitine produces an enhanced effect as compared to either drug administered alone, or simultaneously. This effect is indicative of a synergistic interaction between the two components.

**Claims**

1. A combination comprising docetaxel, or a derivative thereof, and roscovitine.

2. A pharmaceutical composition comprising a combination according to claim 1 and a pharmaceutically acceptable carrier, diluent or excipient.

3. Use of a combination according to claim 1 or claim 2 in the preparation of a medicament for the treatment of a proliferative disorder.

4. A pharmaceutical product comprising docetaxel, or a derivative thereof, and roscovitine, as a combined preparation for simultaneous, sequential or separate use in therapy.

5. A pharmaceutical product according to claim 4 in the form of a pharmaceutical composition comprising a pharmaceutically acceptable carrier, diluent or excipient.

6. A pharmaceutical product according to claim 4 or claim 5 for use in the treatment of a proliferative disorder.

7. A pharmaceutical product according to claim 6 wherein the proliferative disorder is cancer.

8. A pharmaceutical product according to claim 7 wherein the cancer is prostate cancer.

9. Use of roscovitine in the preparation of a medicament for the treatment of a proliferative disorder, wherein said treatment comprises administering to a subject simultaneously, sequentially or separately docetaxel, or a derivative thereof, and roscovitine.

10. Use of roscovitine and docetaxel, or a derivative thereof, in the preparation of a medicament for treating a proliferative disorder.

11. Use of roscovitine in the preparation of a medicament for the treatment of a proliferative disorder, wherein said medicament is for use in combination therapy with docetaxel, or a derivative thereof.

12. Use of docetaxel, or a derivative thereof, in the preparation of a medicament for the treatment of a proliferative disorder, wherein said medicament is for use in combination therapy with roscovitine.

**13.** Use according to any of claims 9 to 12 wherein the proliferative disorder is cancer.

**14.** Use according to claim 13 wherein the cancer is prostate cancer.

**Patentansprüche**

**1.** Kombination, welche Docetaxel oder ein Derivat davon und Roscovitin umfaßt.

**2.** Pharmazeutische Zusammensetzung, welche eine Kombination nach Anspruch 1 und einen pharmazeutisch verträglichen Träger, ein Verdünnungsmittel oder einen Hilfsstoff umfaßt.

**3.** Verwendung einer Kombination nach Anspruch 1 oder Anspruch 2 bei der Herstellung eines Medikaments zur Behandlung einer proliferativen Störung.

**4.** Pharmazeutisches Produkt, welches Docetaxel oder ein Derivat davon und Roscovitin umfaßt, als kombiniertes Präparat für die gleichzeitige, aufeinanderfolgende oder separate Verwendung in der Therapie.

**5.** Pharmazeutisches Produkt nach Anspruch 4 in Form einer pharmazeutischen Zusammensetzung, welche einen pharmazeutisch verträglichen Träger, ein Verdünnungsmittel oder einen Hilfsstoff umfaßt.

**6.** Pharmazeutisches Produkt nach Anspruch 4 oder Anspruch 5 zur Verwendung bei der Behandlung einer proliferativen Störung.

**7.** Pharmazeutisches Produkt nach Anspruch 6, wobei die proliferative Störung Krebs ist.

**8.** Pharmazeutisches Produkt nach Anspruch 7, wobei der Krebs Prostatakrebs ist.

**9.** Verwendung von Roscovitin bei der Herstellung eines Medikaments zur Behandlung einer proliferativen Störung, wobei die Behandlung das gleichzeitige, aufeinanderfolgende oder separate Verabreichen von Docetaxel oder eines Derivats davon und Roscovitin an ein Subjekt umfaßt.

**10.** Verwendung von Roscovitin und Docetaxel oder eines Derivats davon bei der Herstellung eines Medikaments zur Behandlung einer proliferativen Störung.

**11.** Verwendung von Roscovitin bei der Herstellung eines Medikaments zur Behandlung einer proliferativen Störung, wobei das Medikament zur Verwendung in der Kombinationstherapie mit Docetaxel oder einem Derivat davon bestimmt ist.

**12.** Verwendung von Docetaxel oder eines Derivats davon bei der Herstellung eines Medikaments zur Behandlung einer proliferativen Störung, wobei das Medikament zur Verwendung in der Kombinationstherapie mit Roscovitin bestimmt ist.

**13.** Verwendung nach einem der Ansprüche 9 bis 12, wobei die proliferative Störung Krebs ist.

**14.** Verwendung nach Anspruch 13, wobei der Krebs Prostatakrebs ist.

**Revendications**

**1.** Association comprenant du docétaxel, ou un de ses dérivés, et de la roscovitine.

**2.** Composition pharmaceutique comprenant une association suivant la revendication 1 et un support, diluant ou excipient pharmaceutiquement acceptable.

**3.** Utilisation d'une association suivant la revendication 1 ou la revendication 2, dans la préparation d'un médicament destiné au traitement d'un trouble prolifératif.

**4.** Produit pharmaceutique comprenant du docétaxel, ou un de ses dérivés, et de la roscovitine, sous forme d'une préparation combinée pour une utilisation simultanée séquentielle ou séparée en thérapie.

**5.** Produit pharmaceutique suivant la revendication 4, sous forme d'une composition pharmaceutique comprenant un support, diluant ou excipient pharmaceutiquement acceptable.

**6.** Produit pharmaceutique suivant la revendication 4 ou la revendication 5, destiné à être utilisé dans le traitement d'un trouble prolifératif.

**7.** Produit pharmaceutique suivant la revendication 6, dans lequel le trouble prolifératif est le cancer.

**8.** Produit pharmaceutique suivant la revendication 7, dans lequel le cancer est le cancer de la prostate.

**9.** Utilisation de roscovitine dans la préparation d'un médicament destiné au traitement d'un trouble prolifératif, dans laquelle ledit traitement comprend l'administration à un sujet, simultanément, séquentiellement ou séparément, de docétaxel, ou d'un de ses dérivés, et de roscovitine.

**10.** Utilisation de roscovitine et de docétaxel, ou d'un de ses dérivés, dans la préparation d'un médicament destiné au traitement d'un trouble prolifératif.

**11.** Utilisation de roscovitine dans la préparation d'un médicament destiné au traitement d'un trouble prolifératif, dans laquelle ledit médicament est destiné à être utilisé dans une thérapie combinée avec du docétaxel, ou un de ses dérivés.

**12.** Utilisation de docétaxel, ou d'un de ses dérivés, dans la préparation d'un médicament destiné au traitement d'un trouble prolifératif, dans laquelle ledit médicament est destiné à être utilisé dans une thérapie combinée avec de la roscovitine.

**13.** Utilisation suivant l'une quelconque des revendications 9 à 12, dans laquelle le trouble prolifératif est le cancer.

**14.** Utilisation suivant la revendication 13, dans laquelle le cancer est le cancer de la prostate.

**FIGURE 1**

**FIGURE 2**

A

B

FIGURE 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **N. GRAY ; L. DÉTIVAUD ; C. DOERIG ; L. MEIJER.** *Curr. Med. Chem.,* 1999, vol. 6, 859
- **BERGE et al.** *J Pharm Sci,* 1977, vol. 66, 1-19
- *Life Sci.,* vol. 31, 3071-3078
- Relevance of Tumor Models for Anticancer Drug Development. Contrib. Oncol. 1999, vol. 54, 145-156
- Human tumor xenografts: Predictivity, characterization, and discovery of new anticancer agents. **FIEBIG HH ; DENGLER WA ; ROTH T.** Contrib. Oncol. 1999, vol. 54, 29-50
- **DENGLER WA ; SCHULTE J ; BERGER DP et al.** Development of a propidium iodide fluorescence assay for proliferation and cytotoxicity assay. *Anti-Cancer Drugs,* 1995, vol. 6, 522-532
- **GRECO et al.** The search for synergy; A critical review from a response surface perspective. *Pharmacol; Review,* 1995, vol. 47, 331-385
- **LASKA et al.** Simple designs and model-free tests for synergy. *Biometrics,* 1994, vol. 50, 834-841